Europäisches Patentamt
European Patent Office
Office européen des brevets

(19)

(11) Veröffentlichungsnummer: **0 189 870**
**A1**

(12)

# EUROPÄISCHE PATENTANMELDUNG

(21) Anmeldenummer: 86100962.9

(22) Anmeldetag: 24.01.86

(51) Int. Cl.⁴: C 07 D 471/04
A 61 K 31/505
//(C07D471/04, 239:00, 221:00)

(30) Priorität: 26.01.85 DE 3502590

(43) Veröffentlichungstag der Anmeldung:
06.08.86 Patentblatt 86/32

(84) Benannte Vertragsstaaten:
AT CH DE FR GB IT LI

(71) Anmelder: GÖDECKE AKTIENGESELLSCHAFT
Salzufer 16
D-1000 Berlin 10(DE)

(72) Erfinder: Kleinschroth, Jürgen, Dr.
Freiburger Strasse 13
D-7809 Denzlingen(DE)

(72) Erfinder: Mannhardt, Karl, Dr.
Pfauenstrasse 14
D-7807 Elzach-Oberprechtal(DE)

(72) Erfinder: Hartenstein, Johannes, Dr.
Fohrenbühl 23
D-7801 Stegen-Wittental(DE)

(72) Erfinder: Osswald, Hartmut, Prof.Dr.
Kiefernweg 1
D-7808 Waldkirch 2(DE)

(72) Erfinder: Wagner, Bernd, Dr.
Am Lossele 5
D-7809 Denzlingen(DE)

(54) **5-Alkoxy-pyrido ]4,3-d[ pyrimidine Derivate, Verfahren zu deren Herstellung und diese enthaltende Arzneimittel.**

(57) Die Erfindung betrifft neue 5-Alkoxy-pyrido [4,3-d]pyrimidin-Derivate der allgemeinen Formel Ia

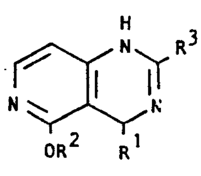

(Ia)

bzw. deren tautomere Formen der allgemeinen Formeln Ib und Ic

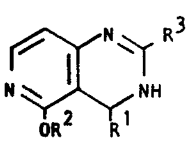

(Ib)

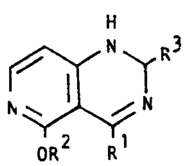

(Ic)

oder aus den Gleichgewichten dieser Formen bestehende Verbindungen, in welchen

$R^1$ einen unsubstituierten oder substituierten Phenylrest

$R^2$ eine geradkettige oder verzweigte Alkylgruppe mit bis zu 6 Kohlenstoffatomen,

$R^3$ eine geradkettige oder verzweigte Alkylgruppe mit bis zu 6 Kohlenstoffatomen oder einen unsubstituierten oder substituierten Phenylrest bedeutet,

deren pharmakologisch unbedenkliche Salze, sowie ein chemisch eigenartiges Verfahren zu deren Herstellung. Die neuen Verbindungen werden als Wirkstoffe in Arzneimitteln zur Bekämpfung cerebraler, cardialer oder peripherer Gefäßerkrankungen oder von stenotischen Erscheinungen eingesetzt.

EP 0 189 870 A1

Croydon Printing Company Ltd.

0189870

## BESCHREIBUNG

Die Erfindung betrifft neue 5-Alkoxy-pyrido[4,3-d]pyrimidin-Derivate der allgemeinen Formel Ia

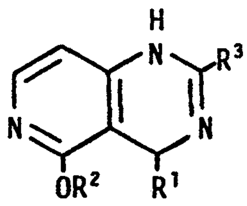 (Ia)

bzw. deren tautomere Formen der allgemeinen Formeln Ib und Ic

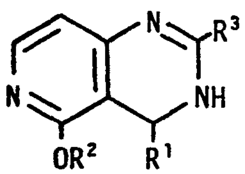 (Ib) 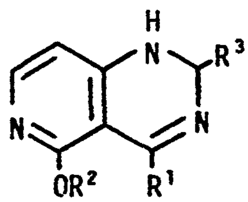 (Ic)

oder aus den Gleichgewichten dieser Formen bestehende Verbindungen, in welchen

$R^1$ einen unsubstituierten oder substituierten Phenylrest

$R^2$ eine geradkettige oder verzweigte Alkylgruppe mit bis zu 6 Kohlenstoffatomen,

$R^3$ eine geradkettige oder verzweigte Alkylgruppe mit bis zu 6 Kohlenstoffatomen oder einen unsubstituierten oder substituierten Phenylrest bedeuten

sowie gegebenenfalls deren pharmakologisch unbedenkliche Salze.

Bevorzugt werden 5-Alkoxy-pyrido[4,3-d]pyrimidin-Derivate der allgemeinen Formeln Ia, b, c
in welchen

$R^1$ einen unsubstituierten oder in 2 und/oder 3 Position substituierten Phenylrest,

$R^2$ eine $C_1$ - $C_4$-Alkylgruppe und

$R^3$ eine $C_1$ - $C_4$-Alkylgruppe oder einen Phenylrest

bedeuten.

Ganz besonders bevorzugt werden 5-Alkoxy-pyrido[4,3-d]pyrimidin-Derivate der allgemeinen Formeln Ia bzw. Ib und Ic in welchen

$R^1$ einen unsubstituierten oder einen durch Halogen, Nitro, Methyl, Methoxy, Difluormethoxy, Trifluormethoxy, Dimethyl- oder Diethylamino, Methylthio, oder Trifluormethyl substituierten Phenylrest oder einen durch Methoxy oder Methylendioxyreste oder durch Halogenatome, die gleich oder verschieden sein können, disubstituierten Phenylrest,

$R^2$ einen Methyl-, Ethyl-, n-Propyl- oder Isopropylrest und

$R^3$ einen Methyl-, Ethyl-, n-Propyl-, Isopropyl- oder Phenylrest

bedeuten.

Die Substitution erfolgt bevorzugt in 2 und/oder 3 Position oder in 2 und/oder 6 Position.

Die Erfindung betrifft weiterhin ein Verfahren zur Herstellung von 5-Alkoxy-pyrido[4,3-d]pyrimidin-Derivaten der allgemeinen Formeln Ia bzw. Ib und Ic, das dadurch gekennzeichnet ist, daß man ein Dihydropyrimidin-Derivat der allgemeinen Formeln IIa

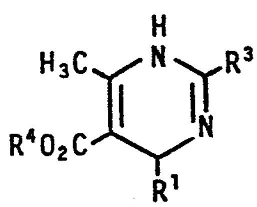

(IIa)

bzw. IIb und IIc

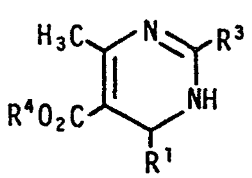

(IIb)

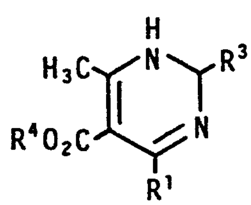

(IIc)

in welchen $R^1$ und $R^3$ die oben angegebene Bedeutung besitzen und $R^4$ eine Methyl- oder Ethylgruppe bedeutet oder aus den Gleichgewichten dieser Formen bestehende Verbindungen, in Gegenwart einer Base mit s-Triazin umsetzt und die so erhaltenen Verbindungen der allgemeinen Formeln IIIa

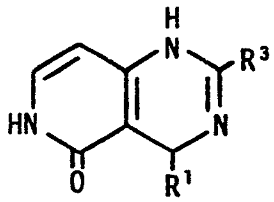

(IIIa)

bzw. IIIb und IIIc

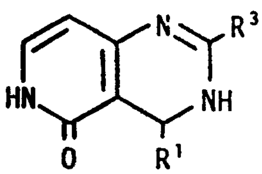

(IIIb)

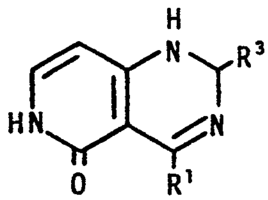

(IIIc)

in denen $R^1$ und $R^3$ die oben angegebene Bedeutung haben oder aus den Gleichgewichten dieser Formen bestehende Verbindungen, zur Einführung des Restes $R^2$ in an sich bekannter Weise O-alkyliert und die erhaltenen Verbindungen der allgemeinen Formeln Ia, b, c anschließend gewünschtenfalls in deren pharmakologisch verträgliche Salze überführt.

Die Verbindungen der allgemeinen Formeln IIa bzw. IIb und IIc sind literaturbekannt (DE-OS 32 34 684) oder können in analoger Weise hergestellt werden.

Zur Durchführung der chemisch eigenartigen Reaktion wird das Dihydropyrimidinderivat mit s-Triazin in einem inerten organischen Lösungsmittel in Gegenwart starker Basen wie z.B. Alkalialkoholaten oder Natriumhydrid auf Temperaturen von 50-160°C, vorzugsweise jedoch 100-150°C, erhitzt. Als Solventien eignen sich hier vor allem polare Lösungsmittel wie Dimethylsulfoxid, Dimethylformamid oder Ethylenglykoldimethylether.

Die Herstellung der 5-Alkoxy-pyrido[4,3-d]pyrimidin-Derivate der allgemeinen Formeln Ia bzw. Ib und Ic erfolgt erfindungsgemäß nach üblichen, für die O-Alkylierung von Lactamen in der Literatur beschriebenen Verfahren (vgl. Adv. Heterocyclic Chem. 12 (1970), 185-212). Geeignete Alkylierungsmittel sind Alkylhalogenide und Alkylsulfonate, Dialkylsulfate und Trialkyloxoniumsalze.

Zur Reaktion mit Alkylhalogeniden werden die Verbindungen der allgemeinen Formeln IIIa bzw. IIIb und IIIc in Form ihrer Metallsalze, vorzugsweise ihrer Alkali- oder Silbersalze eingesetzt, die entweder separat hergestellt oder in situ mit Hilfe geeigneter Basen wie Metallhydriden, -carbonaten oder -alkoxiden in einem aprotischen Lösungsmittel erzeugt werden.

Als geeignete Lösungsmittel kommen, in Abhängigkeit vom jeweiligen Alkylierungsmittel, nahezu alle inerten organischen Lösungsmittel in Frage wie offenkettige, cyclische oder auch aromatische Kohlenwasserstoffe, z.B. n-Pentan, n-Hexan, Cyclohexan, Benzol oder Toluol, halogenierte Kohlenwasserstoffe wie Dichlormethan und 1,2-Dichlorethan, Ether wie z.B. Diethylether und 1,2-Dimethoxyethan, sowie dipolare aprotische Lösungsmittel wie Dimethylformamid, Hexamethylphosphorsäuretriamid und Dimethylsulfoxid. Der Temperaturbereich kann je nach verwendetem Lösungsmittel zwischen -20°C und dem Siedepunkt des betreffenden Lösungsmittel variiert werden.

Aufgrund des ambidenten Charakters des Lactamanions erhält man bei der Alkylierung je nach Reaktionsbedingungen und verwendetem Alkylierungsmittel vielfach Gemische aus O- und N-Alkylierungsprodukten [J. Org. Chem. 32 (1967), 4040 ff].

Die Trennung der erhaltenen Produktgemische kann durch übliche chromatographische Methoden und/oder Kristallisation erfolgen.

Die 5-Alkoxy-pyrido[4,3-d]pyrimidin-Derivate der allgemeinen Formeln Ia bzw. Ib und Ic werden bevorzugt durch Umsetzung der 5-Oxo-pyrido[4,3-d]pyrimidine der allgemeinen Formeln IIIa bzw. IIIb und IIIc mit Trialkyloxoniumsalzen, insbesondere Trimethyloxoniumtetrafluoroborat, in einem aprotischen Lösungsmittel erhalten. Die Herstellung der O-Isopropylverbindungen erfolgt dagegen vorteilhaft durch Alkylierung der Alkalimetallsalze mit Isopropylhalogeniden.

In Abhängigkeit von den Substituenten $R^1$ und $R^3$ weisen Verbindungen der allgemeinen Formeln Ia bzw. Ib und Ic am Pyrimidinring mehr oder minder basischen Charakter auf und werden deshalb zum Zwecke der Reinigung und aus galenischen Gründen bevorzugt in kristalline, pharmakologisch verträgliche Salze übergeführt. Diese werden in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure oder Bernsteinsäure in Frage.

Da die erfindungsgemäßen Verbindungen der allgemeinen Formeln Ia bzw. Ib und Ic entweder an C-2 oder an C-4 ein chirales Zentrum aufweisen, können sie entweder als racemische Gemische oder in Form der Enantiomeren vorliegen.

Die Verbindungen der allgemeinen Formeln Ia bzw. Ib und Ic zeigen als Calciumantagonisten gefäßspasmolytische, vasodilatatorische und antihypertensive Wirkungen.

Aufgrund ihrer gefäßspasmolytischen Wirkungen sind sie vor allem bei cerebralen, cardialen und peripheren Gefäßerkrankungen wie myokardialer Ischämie, bei cerebralem Infarkt, pulmonalen Thrombosen und bei Arteriosklerose und anderen stenotischen Erscheinungen indiziert. Die 5-Alkoxy-pyrido[4,3-d]pyrimidin-Derivate der vorliegenden Erfindung sind daher wertvolle Mittel für die Bekämpfung der Herz-Kreislauf-Mortalität. Ein weiterer Gegenstand der vorliegenden Erfindung ist daher die Verwendung der 5-Alkoxy-pyrido[4,3-d]pyrimidine der allgemeinen Formeln Ia bzw. Ib und Ic bei der Bekämpfung von Gefäßerkrankungen.

Die erfindungsgemäßen Verbindungen der allgemeinen Formeln Ia bzw. Ib und Ic können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citrat-Puffer, Ethanol, Komplexbildner (wie Äthylendiamin-tetraessigsäure und deren nichttoxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süßstoffe enthalten.

Die enteral verabreichten Einzeldosen liegen im Bereich von etwa 5 bis 250 mg, vorzugsweise 10 bis 100 mg. Parenteral werden etwa 1 bis 20 mg gegeben.

Die folgenden Beispiele sollen die Erfindung näher erläutern:

## Beispiel 1

(±)-1,4-Dihydro-5-isopropoxy-2-methyl-4-phenyl-pyrido[4,3-d]-pyrimidin · Hydrochlorid

Zu einer gerührten Suspension von 1,24 g (41,3 mMol) Natriumhydrid (80%ig in Öl) in 50 ml trockenem Dimethylformamid werden 8,97 g (37,5 mMol) (±)-1,4,5,6-Tetrahydro-2-methyl-5-oxo-4-phenyl-pyrido-[4,3-d]pyrimidin portionsweise fest zugegeben. Bei Nachlassen der Gasentwicklung wird 30 Minuten bei Raumtemperatur gerührt; anschließend werden 9,56 g (56,2 mMol) Isopropyliodid in 20 ml Dimethylformamid zugetropft.

Es wird 72 Stunden bei Raumtemperatur gerührt, das Lösungsmittel im Vakuum abgezogen und der Rückstand mit 100 ml Wasser verrührt. Das Wasser wird abdekantiert, der Rückstand in Dichlormethan gelöst und die Lösung erneut mit Wasser gewaschen. Die Dichlormethanlösung wird über Natriumsulfat getrocknet und im Vakuum eingedampft. Der Rückstand wird an Kieselgel mit Ethylacetat/Methanol, $NH_3$ ges. 95:5 chromatographiert.

Die Fraktion mit dem $R_f$ 0,3 wird in Ether gelöst und mit der berechneten Menge Chlorwasserstoff in Ethylacetat in das Hydrochlorid übergeführt und dieses aus Isopropanol/Diisopropylether umkristallisiert. Man erhält das Hydrochlorid des (±)-1,4-Dihydro-5-isopropoxy-2-methyl-4-phenyl-pyrido[4,3-d]pyrimidins in Form farbloser Kristalle vom Schmp. 221°C (Z).

Als weiteres Produkt wird bei der obigen Chromatographie (±)-1,4,5,6-Tetrahydro-6-isopropyl-2-methyl-5-oxo-4-phenyl-pyrido-[4,3-d]pyrimidin ($R_f$ 0,1) isoliert.

Das als Ausgangsmaterial verwendete (±)-1,4,5,6-Tetrahydro-2-methyl-5-oxo-4-phenyl-pyrido[4,3-d]pyrimidin wird wie folgt hergestellt:

(±)-1,4,5,6-Tetrahydro-2-methyl-5-oxo-4-phenyl-pyrido[4,3-d]pyrimidin

Zu einer gerührten Suspension von 4,6 g (0,15 Mol) Natriumhydrid (80%ig in Öl) in 60 ml trockenem Dimethylformamid wird unter Stickstoffatmosphäre eine Lösung von 35,9 g (0,14 Mol) (±)-1,4-Dihydro-2,6-dimethyl-4-phenyl-pyrimidin-5-carbonsäureethylester in 100 ml Dimethylformamid getropft. Bei Nachlassen der Gasentwicklung wird noch 30 Minuten bei Raumtemperatur gerührt; anschließend werden 11,3 g (0,14 Mol) s-Triazin in 60 ml Dimethylformamid zugetropft. Die Reaktionsmischung wird 16 Stunden auf 110°C erhitzt und nach dem Abkühlen im Vakuum eingeengt. Der dunkle Rückstand wird mit 900 ml Aceton im Ultraschallbad behandelt und vom Ungelösten filtriert. Die Acetonlösung wird im Vakuum eingedampft und der Rückstand an Kieselgel mit Dichlormethan/Methanol 3:1 chromatographiert. Die Fraktion mit dem $R_f$ 0,2 wird isoliert und aus Ethylacetat/Methanol umkristallisiert.

Man erhält (±)-1,4,5,6-Tetrahydro-2-methyl-5-oxo-4-phenyl-pyrido-[4,3-d]pyrimidin in Form beiger Kristalle vom Schmp. 263-264°C.

0189870

In analoger Weise werden die folgenden Verbindungen erhalten:

(±)-4-(3-Chlorphenyl)-1,4-dihydro-5-isopropoxy-2-phenyl-pyrido-[4,3-d]pyrimidin (1.a)
Schmp. 78-82°C aus Methanol

(±)-1,4-Dihydro-5-isopropoxy-4-(3-nitrophenyl)-2-phenyl-pyrido-[4,3-d]pyrimidin (1.b)
Schmp. 112-114°C aus Diisopropylether/Ethylacetat

(±)-1,4-Dihydro-5-isopropoxy-2-methyl-4-(2-trifluormethylphenyl)-pyrido[4,3-d]pyrimidin · Hydrochlorid (1.c)
Schmp. 148-150°C aus Diisopropylether/Isopropanol

(±)-1,4-Dihydro-5-isopropoxy-2-(3-nitrophenyl)-4-(2-trifluormethylphenyl)-pyrido[4,3-d]pyrimidin (1.d)

(±)-1,4-Dihydro-5-isopropoxy-2-methyl-4-(2-nitrophenyl)-pyrido[4,3-d]pyrimidin (1.e)

(±)-1,4-Dihydro-5-isopropoxy-2-phenyl-4-(2-trifluormethylphenyl)-pyrido[4,3-d]pyrimidin (1.f)

(±)-1,4-Dihydro-5-isopropoxy-2-(2-trifluormethylphenyl)-4-(2-trifluormethylphenyl)-pyrido[4,3-d]pyrimidin (1.g)

0189870

## Beispiel 2

(±)-1,4-Dihydro-5-methoxy-4-(3-nitrophenyl)-2-phenyl-pyrido-[4,3-d]pyrimidin

3,5 g (10 mMol) (±)-1,4,5,6-Tetrahydro-4-(3-nitrophenyl)-5-oxo-2-phenyl-pyrido[4,3-d]pyrimidin und 3,0 g (20 mMol) Trimethyloxonium-tetrafluoroborat werden in 100 ml 1,2-Dichlorethan unter Stickstoffatmosphäre 3 Tage bei Raumtemperatur gerührt. Es wird zweimal mit 50 ml gesättigter Natriumhydrogencarbonatlösung ausgeschüttelt, die organische Phase abgetrennt, über Natriumsulfat getrocknet und im Vakuum eingedampft.
Der Rückstand wird an Kieselgel mit Toluol/Ethylacetat 3:1 chromatographiert und die Fraktion mit dem $R_f$ 0,35 aus n-Hexan/Diisopropylether umkristallisiert. Man erhält das Produkt in Form blaßgelber Kristalle vom Schmp. 75-80°C.

I. Patentansprüche für die Vertragsstaaten CH, DE, FR, GB, IT

1. 5-Alkoxy-pyrido[4,3-d]pyrimidin-Derivate der allgemeinen Formel Ia

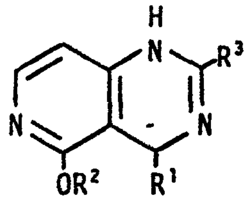

(Ia)

bzw. deren tautomere Formen der allgemeinen Formeln Ib und Ic

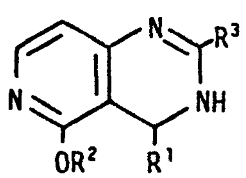

(Ib)

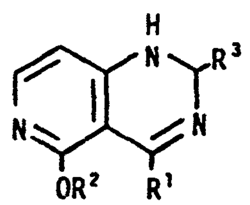

(Ic)

oder aus den Gleichgewichten dieser Formen bestehende Verbindungen, in welchen

$R^1$ einen unsubstituierten oder substituierten Phenylrest

$R^2$ eine geradkettige oder verzweigte Alkylgruppe mit bis zu 6 Kohlenstoffatomen,

$R^3$ eine geradkettige oder verzweigte Alkylgruppe mit bis zu 6 Kohlenstoffatomen oder einen unsubstituierten oder substituierten Phenylrest bedeutet,

sowie deren pharmakologisch unbedenkliche Salze.

2. 5-Alkoxy-pyrido[4,3-d]pyrimidin-Derivate gemäß Anspruch 1, worin

$R^1$ einen unsubstituierten oder in 2 und/oder 3 oder 6 Position mono- oder disubstituierten Phenylrest,

$R^2$ eine $C_1$ - $C_4$-Alkylgruppe und

$R^3$ eine $C_1$ - $C_4$-Alkylgruppe oder einen Phenylrest

bedeutet.

3. 5-Alkoxy-pyrido[4,3-d]pyrimidin-Derivate gemäß Anspruch 1 und 2, worin

$R^1$ einen unsubstituierten oder einen durch Halogen, Nitro, Methyl, Methoxy, Difluormethoxy, Trifluormethoxy, Dimethylamino, Diethylamino, Methylthio, oder Trifluormethyl substituierten Phenylrest oder einen durch Methoxy oder Methylendioxyreste oder Halogenatome, die gleich oder verschieden sein können, disubstituierten Phenylrest,

$R^2$ einen Methyl-, Ethyl-, n-Propyl- oder Isopropylrest und

$R^3$ einen Methyl-, Ethyl-, n-Propyl-, Isopropyl- oder Phenylrest

bedeutet.

4. Verfahren zur Herstellung von 5-Alkoxy-pyrido[4,3-d]pyrimidin-Derivaten gemäß Anspruch 1 bis 3, dadurch gekennzeichnet, daß man ein Dihydropyrimidin-Derivat der allgemeinen Formeln IIa,b,c

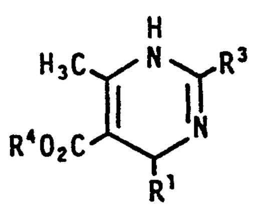

(IIa)

bzw. IIb und IIc

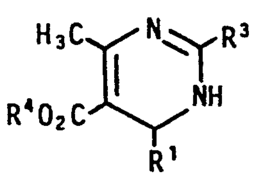

(IIb)

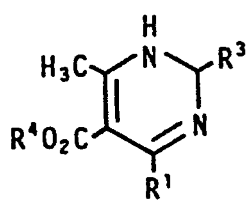

(IIc)

in welchen die Reste $R^1$ und $R^3$ und die in den jeweiligen Ansprüchen angegebene Bedeutung haben und $R^4$ eine Methyl oder Ethylgruppe bedeutet,

oder aus den Gleichgewichten dieser Formen bestehende Verbindungen in Gegenwart einer Base mit s-Triazin umsetzt und die so erhaltenen Verbindungen der allgemeinen Formeln IIIa,

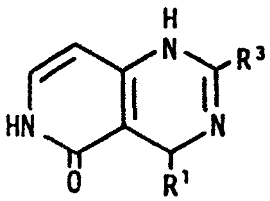
(IIIa)

bzw. IIIb und IIIc

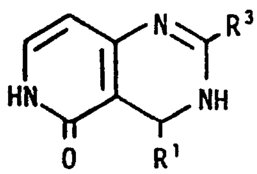
(IIIb)

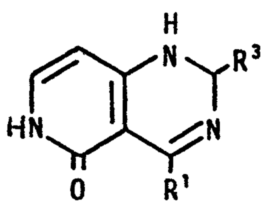
(IIIc)

in denen $R^1$ und $R^3$ die oben angegebene Bedeutung haben oder aus den Gleichgewichten dieser Formen bestehende Verbindungen, in an sich bekannter Weise O-alkyliert und die erhaltenen Verbindungen der allgemeinen Formeln Ia, b, c anschließend gewünschtenfalls in deren pharmakologisch verträgliche Salze überführt.

5. Arzneimittel enthaltend 5-Alkoxy-pyrido[4,3-d]pyrimidin-Derivate gemäß Anspruch 1 bis 3 zur Bekämpfung cerebraler, cardialer oder peripherer Gefäßerkrankungen oder von stenotischen Erscheinungen.

0189870

II. Patentansprüche für Vertragsstaat AT

1. Verfahren zur Herstellung von 5-Alkoxy-pyrido[4,3-d]pyrimidin-Derivaten der allgemeinen Formel Ia

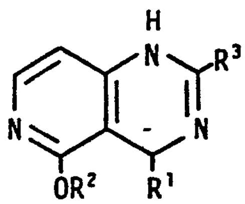

(Ia)

bzw. von deren tautomeren Formen der allgemeinen Formeln Ib und Ic

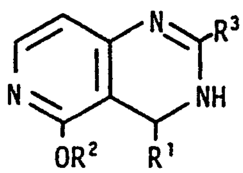

(Ib)

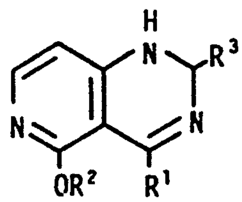

(Ic)

oder von aus den Gleichgewichten dieser Formen bestehenden Verbindungen, in welchen

$R^1$ einen unsubstituierten oder substituierten Phenylrest

$R^2$ eine geradkettige oder verzweigte Alkylgruppe mit bis zu 6 Kohlenstoffatomen,

$R^3$ eine geradkettige oder verzweigte Alkylgruppe mit bis zu 6 Kohlenstoffatomen oder einen unsubstituierten oder substituierten Phenylrest bedeutet,

sowie von deren pharmakologisch unbedenklichen Salzen, dadurch gekennzeichnet, daß man ein Dihydropyrimidin-Derivat der allgemeinen Formeln IIa, b, c

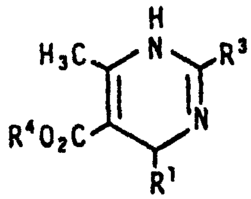

(IIa)

bzw. IIb und IIc

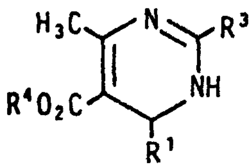

(IIb)

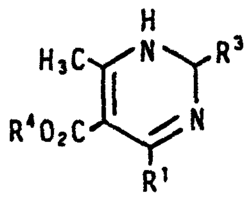

(IIc)

in welchen die Reste $R^1$ und $R^3$ und die in den jeweiligen Ansprüchen angegebene Bedeutung haben und $R^4$ eine Methyl oder Ethylgruppe bedeutet,

oder aus den Gleichgewichten dieser Formen bestehende Verbindungen in Gegenwart einer Base mit s-Triazin umsetzt und die so erhaltenen Verbindungen der allgemeinen Formeln IIIa,

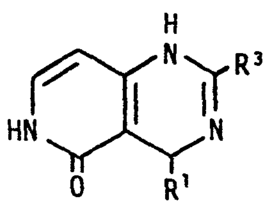

(IIIa)

bzw. IIIb und IIIc

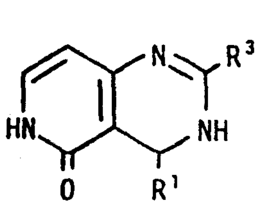

(IIIb)

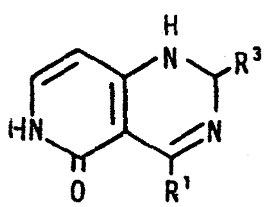

(IIIc)

in denen $R^1$ und $R^3$ die oben angegebene Bedeutung haben oder aus den Gleichgewichten dieser Formen bestehende Verbindungen, in an sich bekannter Weise O-alkyliert und die erhaltenen Verbindungen der allgemeinen Formeln Ia, b, c anschließend gewünschtenfalls in deren pharmakologisch verträgliche Salze überführt.

2. Verfahren gemäß Anspruch 1, dadurch gekennzeichnet, daß

$R^1$ einen unsubstituierten oder in 2 und/oder 3 oder 6 Position mono- oder disubstituierten Phenylrest,

$R^2$ eine $C_1$ - $C_4$-Alkylgruppe und

$R^3$ eine $C_1$ - $C_4$-Alkylgruppe oder einen Phenylrest

bedeutet.

3. Verfahren gemäß Anspruch 1 und 2, dadurch gekennzeichnet, daß

$R^1$ einen unsubstituierten oder einen durch Halogen, Nitro, Methyl, Methoxy, Difluormethoxy, Trifluormethoxy, Dimethylamino, Diethylamino, Methylthio, oder Trifluormethyl substituierten Phenylrest oder einen durch Methoxy oder Methylendioxyreste oder Halogenatome, die gleich oder verschieden sein können, disubstituierten Phenylrest,

$R^2$ einen Methyl-, Ethyl-, n-Propyl- oder Isopropylrest und

$R^3$ einen Methyl-, Ethyl-, n-Propyl-, Isopropyl- oder Phenylrest

bedeutet.

Europäisches Patentamt

# EUROPÄISCHER RECHERCHENBERICHT

0189870

Nummer der Anmeldung

EP 86 10 0962

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | EP-A-0 103 796 (BAYER) * Patentansprüche 1 und 10 * & DE - A - 3 234 684 | 1,5 | C 07 D 471/04<br>A 61 K 31/505//<br>(C 07 D 471/04<br>C 07 D 239:00<br>C 07 D 221:00) |
| | ----- | | |
| | | | RECHERCHIERTE SACHGEBIETE (Int Cl 4) |
| | | | C 07 D 471/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 09-05-1986 | ALFARO I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82